# EUROPEAN PATENT SPECIFICATION

(11) **EP 3 533 395 B1**
(45) Date of publication and mention of the grant of the patent: **16.12.2020**
(21) Application number: 17864537.0
(22) Date of filing: 30.08.2017
(51) Int. Cl.: G01N 33/487

(54) **INTEGRATED BLOOD GLUCOSE MEASURING DEVICE**
INTEGRIERTE BLUTZUCKERMESSVORRICHTUNG
DISPOSITIF INTÉGRÉ DE MESURE DE LA GLYCÉMIE

(30) Priority: 31.10.2016 KR 20160143858
(43) Date of publication of application: 04.09.2019
(73) Proprietor: I-sens, Inc., Seoul 06646 (KR)
(72) Inventor: CHAE, Kyung Chul, Seongnam-si Gyeonggi-do 13589 (KR); CHOI, Hyun Ho, Seoul 03097 (KR); LYU, Goang Yel, Goyang-si Gyeonggi-do 10518 (KR); WANG, Ji Hoon, Goyang-si Gyeonggi-do 10240 (KR); CHA, Geun Sig, Seoul 03610 (KR); NAM, Hak hyun, Seoul 01227 (KR)
(74) Representative: Brann AB
(86) International application number: PCT/KR2017/009505
(87) International publication number: WO 2018/080003

(56) References cited:
- JP-A- 2003 042 994
- KR-A- 20070 073 914
- KR-A- 20110 095 197
- KR-B1- 101 322 460
- US-A1- 2008 299 009
- US-A1- 2010 041 156
- US-A1- 2010 096 403
- US-A1- 2015 144 484

## Description

### TECHNICAL FIELD

The present disclosure relates to an integrated blood glucose measuring device. More particularly, the present disclosure relates to an integrated blood glucose measuring device having a structure accommodating a strip cartridge storing a plurality of test strips therein and allowing the test strips to be supplied and discharged one by one from the strip cartridge by moving a manipulation knob linearly in two separate stages. The integrated blood glucose measuring device can accurately supply and discharge the test strips and only open a supply open area of the strip cartridge when the manipulation knob is being operated, due to a simple operating method and structure thereof. Accordingly, the sealing function with respect to the strip cartridge can be reliably maintained, thereby protecting the test strips from degrading even when used for a long time, so that the accuracy of blood glucose measurement results can be enhanced.

### BACKGROUND ART

Diabetes or diabetes mellitus is a chronic medical condition that is common in modern people. In the Republic of Korea, it affects 2 million people, about 5% of the total population.

Diabetes is caused from the absolute deficiency or relative insufficiency of insulin produced by the pancreas, due to various causes such as obesity, stress, poor eating habits, inherited hereditary factors, and thus the sugar in the blood cannot be balanced, but may be absolutely increased.

The blood usually contains a certain concentration of glucose and tissue cells gain energy therefrom.

However, when glucose is increased excessively, it is not properly stored in the liver, muscles, or adipose tissue and accumulates in the blood. As a result, patients with diabetes maintain a much higher blood sugar level than other people. Excessive blood sugar passes through the tissues and is discharged into the urine, resulting in a deficiency of sugar, which is absolutely necessary for each tissue of the body, thereby causing abnormalities in respective tissues of the body.

Diabetes mellitus is characterized by the absence of subjective symptoms at the beginning. When diabetes progresses, diabetes-specific symptoms such as diarrhea, polyuria, weight loss, general anxiety, skin itchiness, and scarring of the hands and feet are present. Further progression of diabetes leads to complications such as visual disturbances, hypertension, kidney disease, paralysis, periodontal disease, muscle spasms and neuralgia, and gangrene.

Systematic blood glucose measurement and treatment should be performed to diagnose such diabetes beforehand and manage the condition so as not to progress to diabetes and its complications.

For people with diabetes or those who have not yet developed diabetes, but have higher blood sugar than normal, medical device manufacturers offer a variety of blood glucose meters to measure blood glucose at home.

The blood glucose meter is configured such that a test strip is inserted into a casing, a blood sample is absorbed to the test strip, a blood glucose level is measured by inspecting the blood sample using a blood glucose measurement module in the casing, a blood glucose measurement result is calculated using an calculation module, and the blood glucose measurement result is output to a separate display unit.

In order to use such a blood glucose meter, a user must possess and carry a separate strip storage case accommodating a plurality of test strips, take out one test strip from the strip storage case, insert the taken test strip into the blood glucose meter to measure a blood glucose level, discharge the test strip from the blood glucose meter, and then discard the discharged test strip. Thus, the blood glucose meter is inconvenient to use. Along with this inconvenience, the user must carry the test strip storage case separately, which is very inconvenient.

In order to solve the inconvenience in use, various types of integrated type blood glucose meters, in which a strip cartridge containing a plurality of test strips can be inserted into a space inside a blood glucose meter casing and test strips can be supplied one by one from the strip cartridge, have been developed recently.
However, various integrated type blood glucose meters developed at present have various problems, such as the complicated structure or operation method, or insufficient supply of test strips.
US 2008/299009 A1 discloses a disposable glucometer including mechanism to automatically load test strips one at a time from the testing region and eject the strip after test is complete. US 2010/041156 A1 discloses an instrument including a first and a second test-sensor cartridge.
US 2015/144484 A1 discloses a blood glucose monitor including a can, replaceable sensor cartridge.

### DISCLOSURE

### Technical Problem

In accordance with the present invention, there is provided an integrated blood glucose measuring device as defined by claim 1. Further advantageous embodiments of the present invention are defined by the dependent claims. Accordingly, the present invention has been made in consideration of the above-described problems occurring in the related art, and the present disclosure provides an integrated blood glucose measuring device having a structure accommodating a strip cartridge storing a plurality of test strips therein and allowing the test strips to be supplied and discharged one by one from the strip cartridge by moving a manipulation knob linearly in two separate stages, so that the integrated blood glucose measuring device can accurately supply and discharge the test strips, due to a simple operating method and structure thereof.

The present invention also provides an integrated blood glucose measuring device able to only open a supply open area of the strip cartridge when the manipulation knob is being operated, so that the sealing function with respect to the strip cartridge can be reliably maintained, thereby protecting the test strips from degrading even when used for a long time, so that the accuracy of blood glucose measurement results can be enhanced.

### Technical Solution

According to the present invention, an integrated blood glucose measuring device includes: a casing having a structure, by which a strip cartridge accommodating a plurality of test strips in an inner space thereof is interchangeably inserted into the casing, the casing having a strip outlet at one portion thereof, through which a test strip, among the plurality of test strips, is discharged outward; and a strip discharging unit mounted on the casing to be operable by a user using a manipulation knob to discharge the plurality of test strips one by one from the strip cartridge through the strip outlet, wherein the manipulation knob is configured to move linearly in first and second individual linear movements by manipulation of the user, such that during the first linear movement, the test strip is partially moved into a blood glucose measurement mode state through the strip outlet, and during the second linear movement, the test strip is discharged and removed from the strip outlet.

The strip discharging unit may be configured such that the manipulation knob returns to an original position thereof after both the first and second linear movements are performed.

The strip discharging unit includes a manipulation knob module including the manipulation knob and being operated by the user to move linearly; a discharge plate linearly moving integrally with the manipulation knob module to push and discharge the test strip, supplied through the supply open area of the strip cartridge, to the strip outlet; and a cartridge opening/closing module operable to open and close the supply open area of the strip cartridge in association with the linear movement of the manipulation knob module.

The manipulation knob module may be exposed to one surface of the casing to be coupled in a linearly movable manner, wherein the manipulation knob module includes: a push button coupled to the manipulation knob to be elastically supported upward by the manipulation knob in a direction perpendicular to the linear movement direction of the manipulation knob, so as to linearly move integrally with the manipulation knob; and a moving body coupled to the manipulation knob or the push button to linearly move integrally with the manipulation knob or the push button, wherein the casing is provided with a separation movement guide to guide the manipulation knob module such that the manipulation knob module moves linearly in the first and second linear movements.

After the first linear movement of the manipulation knob module is completed, the separation movement guide guides the manipulation knob module such that the second linear movement proceeds while the push button is pressed downward.

The casing may be provided with a movement restricting guide guiding the manipulation knob module to prevent the manipulation knob module from returning to a reference position before the first and second linear movements are completed.

The movement restricting guide may be provided to restrict the manipulation knob module from returning to the original position in a state in which the first linear movement of the manipulation knob module is completed.

The movement restricting guide may include: a movement path guide rail guiding first and second linear movement paths of the manipulation knob module; and a return path guide rail guiding an original return path of the manipulation knob module, wherein the movement path guide rail is provided with a ratchet protrusion to prevent the manipulation knob module from returning to the original position.

The cartridge opening/closing module includes: a rotary sleeve rotating about a longitudinal axis integrally with the linear movement of the manipulation knob module, with an operation protrusion integrally provided on one portion of an outer circumference thereof; and an opening/closing cover arranged to be movable up and down linearly to open/close the supply open area of the strip cartridge and elastically supported by an elastic spring in a direction to contact the rotary sleeve, wherein, as the rotary sleeve rotates, the opening/closing cover is pressed by or released from the operation protrusion to move up and down by an elastic force.

The manipulation knob module may include: a push button coupled to the manipulation knob to be elastically supported upward by the manipulation knob in a direction perpendicular to the linear movement direction of the manipulation knob, so as to linearly move integrally with the manipulation knob; and a moving body coupled to the manipulation knob or the push button to be linearly moved at the same time, wherein the rotary sleeve of the cartridge opening/closing module and the moving body of the manipulation knob module are provided with a cam protrusion and a cam groove mutually engaged with a cam structure, wherein the rotary sleeve is configured to rotate through the cam structure as the moving body moves linearly.

In a state in which the cartridge opening/closing module opens the supply open area of the strip cartridge, the discharge plate may move linearly to push out the test strip so as to close the supply open area of the strip cartridge.

### Advantageous Effects

The integrated blood glucose measuring device according to the present invention has a structure accommodating a strip cartridge storing a plurality of test strips therein and allowing the test strips to be supplied and discharged one by one from the strip cartridge by moving a manipulation knob linearly in two separate stages, so that the integrated blood glucose measuring device can accurately supply and discharge the test strips, due to a simple operating method and structure thereof.

In addition, the integrated blood glucose measuring device can only open a supply open area of the strip cartridge when the manipulation knob is being operated, so that the sealing function with respect to the strip cartridge can be reliably maintained, thereby protecting the test strips from degrading even when used for a long time, so that the accuracy of blood glucose measurement results can be enhanced.

### DESCRIPTION OF DRAWINGS

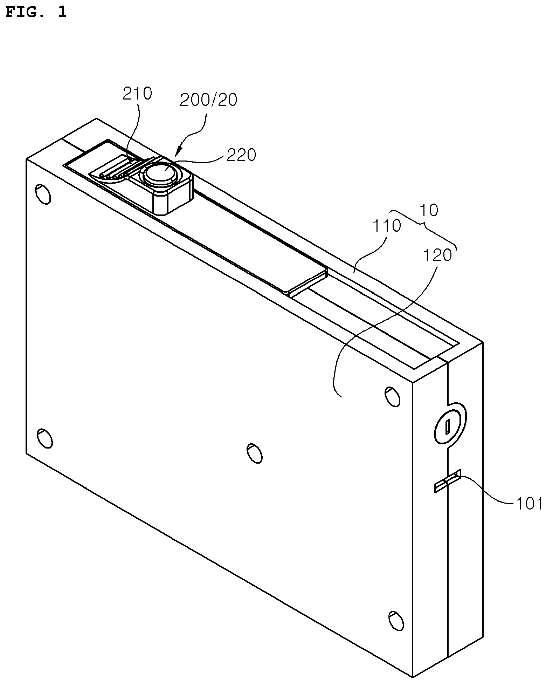

FIG. 1 is a perspective view schematically illustrating an outer shape of an integrated blood glucose measuring device according to an embodiment

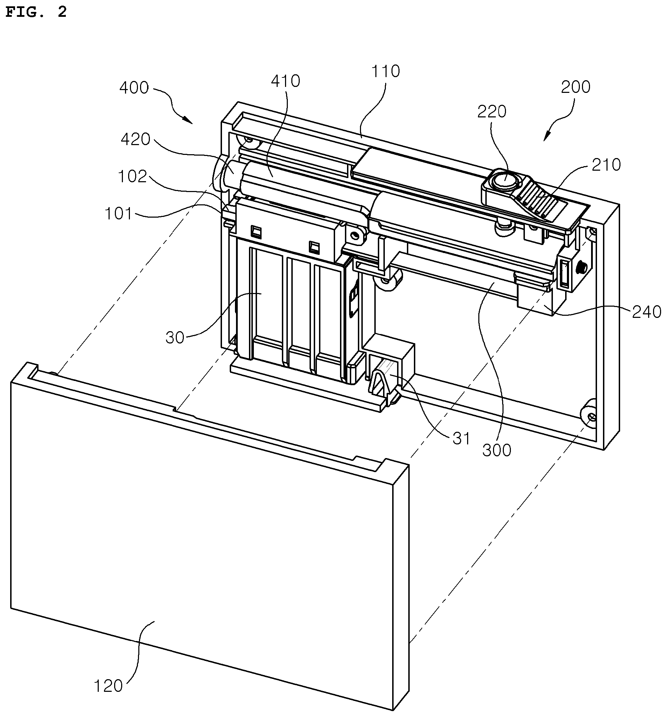

FIG. 2 is a perspective view schematically illustrating an internal assembly structure of the integrated blood glucose measuring device according to an embodiment

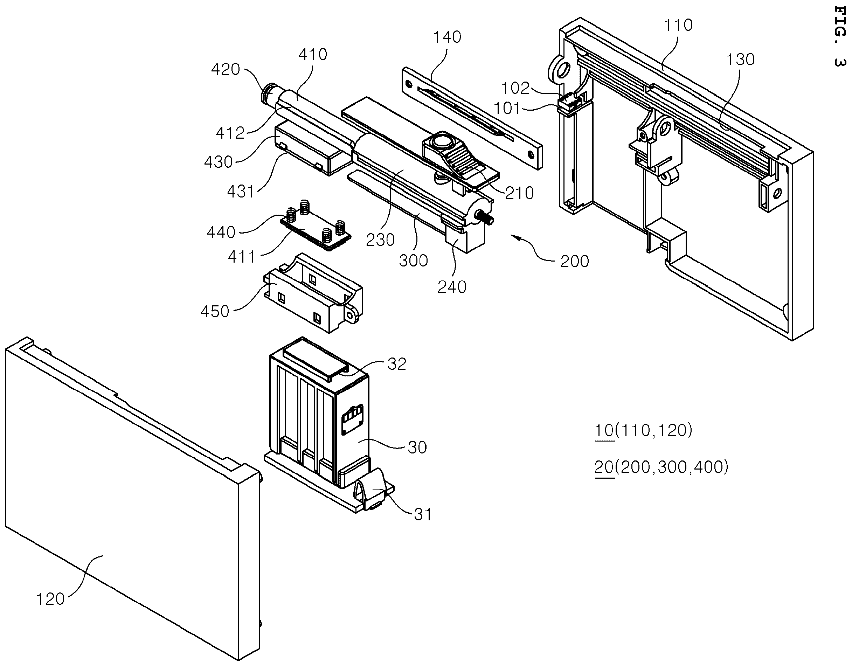

FIG. 3 is an exploded perspective view schematically illustrating a configuration of the integrated blood glucose measuring device according to an embodiment
FIG. 4 is a cross-sectional view schematically illustrating an internal structure of the integrated blood glucose measuring device according to an embodiment
FIG. 5 is an exploded perspective view schematically illustrating a coupling structure of an operation knob module and a cartridge opening/closing module of the integrated blood glucose measuring device according to an embodiment
FIG. 6 is an enlarged cross-sectional view for explaining a guide structure of the operation knob module of the integrated blood glucose measuring device according to the embodiment
FIG. 7 is a view for explaining an operation state of the separation movement guide unit for guiding the operation knob module according to the embodiment
FIG. 8 is a cross-sectional view illustrating an initial movement start state of the operation knob module according to the embodiment
FIG. 9 is a cross-sectional view illustrating a first-stage linear movement state of the operation knob module according to an embodiment and
FIG. 10 is a cross-sectional view illustrating a second-stage linear movement state of the operation knob module according to an embodiment.

### MODE FOR INVENTION

Hereinafter, embodiments of the present invention will be described in detail with reference to the accompanying drawings. Throughout this document, reference should be made to the drawings, in which the same reference numerals and symbols will be used to designate the same or like components. In the following description of the present disclosure, detailed descriptions of known functions and components incorporated herein will be omitted in the case that the subject matter may be rendered unclear thereby.

The integrated blood glucose measuring device is an integrated blood glucose measuring device which can receive a strip cartridge containing a plurality of test strips therein. The integrated blood glucose measuring includes a casing 10 and a strip discharging unit 20

The casing 10 may be an external cover member of the entire device, divided into a casing body 110 and a casing cover 120, such that a storage space is defined therein. The casing 10 is configured such that the strip cartridge 30 can be interchangeably inserted into the inner space thereof.

The strip cartridge 30 is configured such that a plurality of test strips S are stacked in a top-bottom direction therein. The strip cartridge 30 has a supply open area 32 on an upper portion thereof to supply the test strips and an elastic spring (not shown) on a lower portion thereof to push the test strips S upward by the elastic force thereof. Therefore, when one test strip S, among the plurality of test strips S, is discharged through the supply open area 32, the next test strip S, stacked below the discharged strip S, is moved upwards toward the supply open area 32, being a supply standby state.

Since the test strip S accommodated in the strip cartridge 30 tends to be degraded by external humidity, it is preferable that the test strip S is stored in a sealed state. Although not shown, a separate sealing tape (not shown) or the like may be attached to the supply open area 32 such that the test strip may be inserted into the casing 10 after the sealing tape is removed. In addition, it is important that the sealed state of the inner space of the strip cartridge 30 is similarly maintained even when the sealed state is inserted into the casing 10. Such a sealing state can be obtained through an opening/closing cover 430 of a cartridge opening/closing module 400, which will be described later.

The strip cartridge 30 is configured to be replaceable with a new strip cartridge after all the test strips S accommodated therein are used, and may be configured to be easily attached to and detached from the casing 10 using a separate fixing clip 31.

A strip outlet 101 is provided in one portion of the casing 10, so that the test strip S supplied from the strip cartridge 30 can be discharged therethrough.

The strip discharging unit 20 is mounted on the casing 10 to be operable by a user using an operation knob 210 and is operable to discharge the test strips S one by one from the strip cartridge 30 through the strip outlet 101.

At this time, the strip discharging unit 20 is configured such that the operation knob 210 is separately moved in first and second linear movements by the user's manipulation. In the first linear movement, the test strip S is partially discharged through the strip outlet 101 into a position of blood glucose measuring mode, and in the second linear movement, the test strip S is completely discharged and removed from the strip outlet 101.

According to this structure, a user can linearly move the manipulation knob 210 in the first linear movement, thereby partially moving the test strip S from the strip cartridge 30 from the strip outlet 101. At this time, the test strip S is held in contact with a contact pin 102 mounted around the strip outlet 101 of the casing 10. In this state, a user can perform a blood glucose measurement operation by adsorbing a blood sample on the discharged test strip S. When the blood glucose measurement operation is completed as described above, the user has to discharge the test strip S from the casing 10, so that the manipulation knob 210 can be linearly moved in the second linear movement, during which the test strip S is discharged through the strip outlet 101.

Accordingly, the integrated blood glucose measuring device not only can position the test strip S in the blood glucose measurement mode state, but also can discharge and remove the test strip, by the two-stage linear movement of the manipulation knob 210, so that the operation method is simple and the structure can also be simplified.

Further, the strip discharging unit 20 can be provided so that the manipulation knob 210 can be returned to the original position after both the first and second linear movements are performed. This structure prevents the discharging process from being stopped in the middle stage of discharging the test strip S so that the test strip S can be regularly discharged and the damage of the test strip S can be prevented, thereby providing various additional functions such as management of the remaining quantity of the test strips S in the strip cartridge 30.

Next, the configuration of the integrated blood glucose measuring device will be described in detail.

The strip discharging unit 20 includes a manipulation knob module 200 including a manipulation knob 210, which is operative to move linearly by a user's manipulation, a discharge plate 300 linearly moving integrally with the manipulation knob module 200 to push and discharge the test strip S supplied through the supply open area 32 to the strip outlet 101, and a cartridge opening/closing module 400 operating in conjunction with the linear movement of the manipulation knob module 200 to open/close the supply open area 32 of the strip cartridge 30.

According to this structure, when the user operates the manipulation knob 210 to linearly move, the manipulation knob module 200 moves linearly together with the manipulation knob 210, and the discharge plate 300 linearly moves together with the manipulation knob 210. The cartridge opening and closing module 400 keeps the supply open area 32 of the strip cartridge 30 in a closed state. When the manipulation knob module 200 is linearly moved, the cartridge opening and closing module 400 is operated to open the supply open area 32. When the manipulation knob module 200 is moved linearly, the supply open area 32 of the strip cartridge 30 is opened by the cartridge opening/closing module 400 and the discharge plate 300 moves integrally with the manipulation knob module 200 to push out and discharge the test strip S to the strip outlet 101. Here, when the manipulation knob module 200 moves linearly in the first stage, the test strip S is partially discharged from the strip outlet 101 by the discharge plate 300, and in this state, when the manipulation knob module 200 is linearly moved in the second stage, the test strip S is discharged and removed by the discharge plate 300.

The manipulation knob module 200 includes a manipulation knob 210, a push button 220, and a moving body 230. The manipulation knob 210 is provided to be slidably moved by a user, and is exposed and coupled to one surface of the casing 10 to be linearly movable. The push button 220 is coupled to the manipulation knob 210 to pass through the casing 10 in a direction perpendicular to the linear movement direction of the manipulation knob 210, and is coupled to the manipulation knob 210 to be elastically supported upward by a separate elastic spring 224, such that the push button is linearly movable integrally with the manipulation knob 210. The moving body 230 is configured to be coupled with the manipulation knob 210 or the push button 220 so as to linearly move therewith.

The discharge plate 300 is coupled to the moving body 230 using a separate connecting block 240 to move integrally with the manipulation knob module 200.

Here, the casing 10 is provided with the separation movement guide 130 for guiding the manipulation knob module 200 so that the manipulation knob module 200 linearly moves in the first and second stages. After the first linear movement of the manipulation knob module 200 is completed, the separation movement guide 130 guides the manipulation knob module 200 so that the second linear movement proceeds with the push button 220 pressed downward.

In addition, the casing 10 is provided with a movement restricting guide 140 for guiding the manipulation knob module 200 to prevent the manipulation knob module 200 from returning to a reference position before the first and second linear movements are completed. The movement restricting guide 140 is provided to restrict the movement of the manipulation knob module 200 from returning to the original state when the first-stage linear movement of the manipulation knob module 200 is completed.

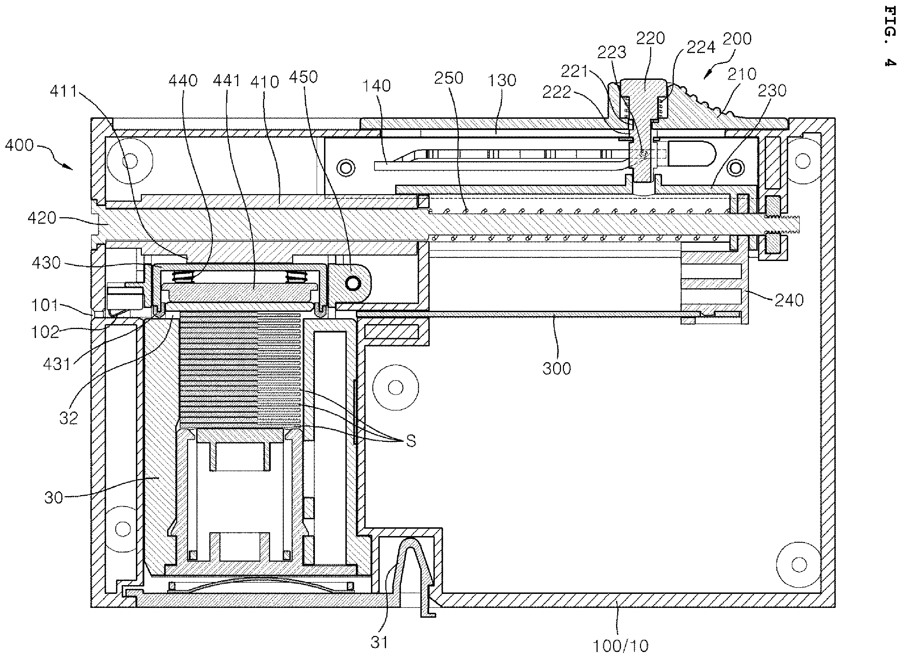

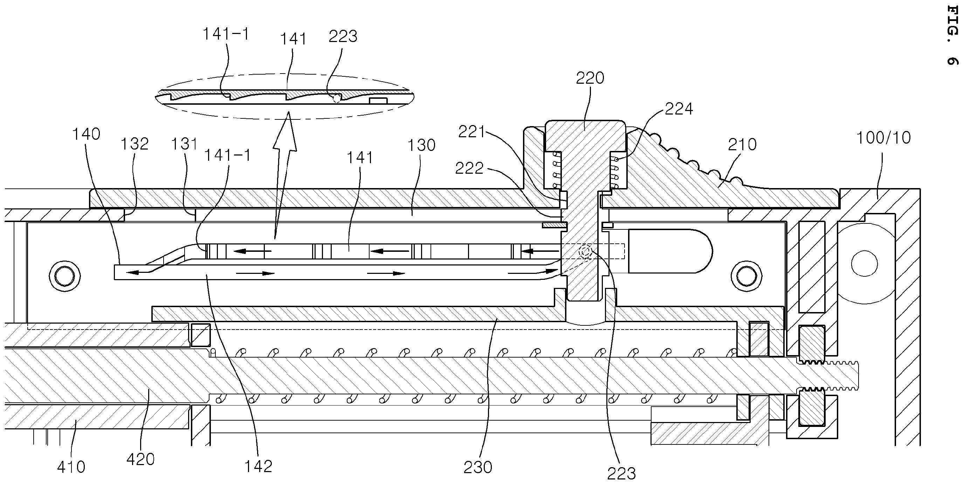

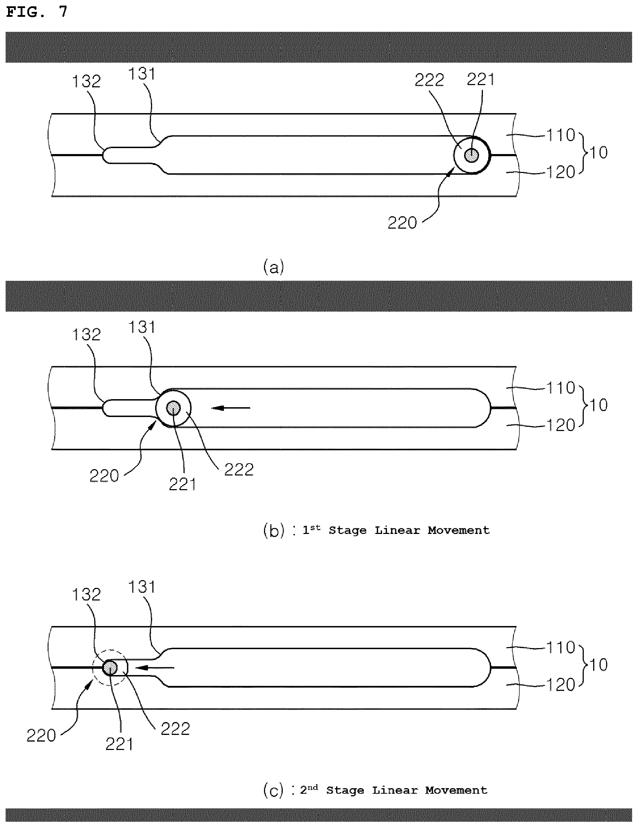

Referring to FIGS. 4, 6 and 7 in more detail, the push button 220 has a relatively large-diameter convex protrusion 222 and a concave depression 221 formed on a lower body portion thereof, alternating along the vertical longitudinal direction, in a state in which the push button 220 is inserted using the manipulation knob 210. As illustrated in FIGS. 6 and 7, the separating movement guide 130 is provided on one surface of the casing 10 to guide the linear movement path of the push button 220. In a location where the first stage linear movement is completed, a stepped portion 131 is formed to engage with the convex projection 222 of the push button 220. In a location where the second stage linear movement is completed, a finishing end 132 is formed to engage with the concave depression 221 of the push button.

The push button 220 is configured such that, in a state in which push button 220 is elastically supported by the elastic spring 224, the convex protrusion 222 is guided along the separation movement guide 130 and linearly moved together with the manipulation knob 210 (see FIG. 7 (a)), and at a point in time at which the first stage linear movement is completed, the convex protrusion 222 abuts against the stepped portion 131 (see FIG. 7 (b)). In this state, when the push button 220 is pressed downward, the convex protrusion 222 and the stepped portion 131 are disengaged from each other, so that the concave depression 221 of the push button 220 is guided along the separation movement guide 130 to linearly move in the second stage linear movement to the section where the concave depression 221 is engaged with the finishing end 132 (see FIG. 7 (c)).

As illustrated in FIGS. 4 and 6, a separate guide protrusion 223 protrudes from a body part of the push button 220. The movement restricting guide 140 guides the movement path of the manipulation knob module 200 and restricts the returning of the manipulation knob module to the original position by the engagement with the guide protrusion 223.

As illustrated in FIG. 6, the movement restricting guide 140 includes a movement path guide rail 141 for guiding the first and second linear movement paths of the manipulation knob module 200, and a return path guide rail 142 for guiding the original return path of the manipulation knob module 200. At this time, the movement path guide rail 141 is provided with a plurality of ratchet protrusions 141-1 in the intermediate section to prevent the manipulation knob module 200 from returning to the original position.

Thus, in the push button 220, as illustrated in FIG. 6, the guide protrusion 223 is moved along the movement path guide rail 141 in the first and second linear motion sections, and after the first and second linear movements are completed, the guide protrusion returns to the original position along the return path guide rail 142. Here, the guide protrusion 223 smoothly moves linearly without engaging with the ratchet protrusions 141-1 during the first and second stage linear movements. However, when there is an attempt to return the guide protrusion 223 to the original position in this section, i.e. during the linear movements, the guide protrusion 223 is engaged with the ratchet protrusion 141-1 so as not to return to the original position, since the ratchet protrusions 141-1 are formed on the movement path guide rail 141. Therefore, once the manipulation knob 210 is started to move linearly in the first stage linear movement, the manipulation knob 210 cannot return to the original state before all the stages up to the second stage linear movement are completed.

On the other hand, the cartridge opening/closing module 400 includes a rotary sleeve 410, which rotates about a longitudinal axis integrally with the linear movement of the manipulation knob module 200, with an operation protrusion 411 integrally formed on one portion of the outer circumference thereof, and an opening/closing cover 430 which is arranged to be movable up and down linearly so as to open/close the supply open area 32 of the strip cartridge 30 and is elastically supported by the elastic spring 440 in a direction of contacting the rotary sleeve 410. At this time, as the rotary sleeve 410 rotates, the opening/closing cover 430 is pressed by or released from the operation protrusion 411 so as to move up and down by an elastic force.

A sealing member 431 may be mounted on a lower edge of the opening/closing cover 430 so as to seal the supply open area 32 of the strip cartridge 30. The elastic spring 440 may be mounted on a separate support plate 441 to elastically support the opening/closing cover 430, and a separate base housing 450 may be disposed on the outer periphery of the opening/closing cover 430 to surround and support these elements.

A separate support shaft 420 may be inserted into the inner space of the rotary sleeve 410 along the central axis direction and the rotary sleeve 410 may be coupled to the outer peripheral surface of the support shaft 420 to rotate.

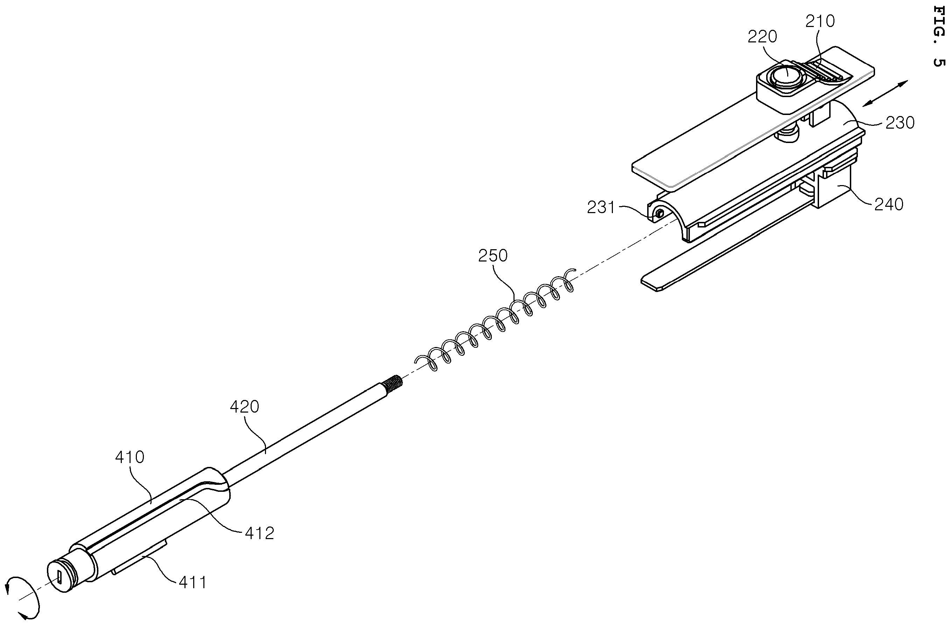

The structure in which the rotary sleeve 410 rotates in conjunction with the linear movement of the manipulation knob module 200 may be accomplished by the configuration including a cam structure in which the moving body 230 of the manipulation knob module 200 and the rotary sleeve 410 are engaged with each other. For example, as illustrated in FIG. 5, a cam groove 412 having a curved path is formed on the outer circumferential surface of the rotary sleeve 410, and a cam protrusion 231 may be formed on one portion of the inner surface of the moving body 230 so as to be engaged with the cam groove 412.

When the moving body 230 of the manipulation knob module 200 is linearly moved according to this structure, the cam protrusion 231 and the cam groove 412 are mutually moved in the engaged state and the rotary sleeve 410 rotates about the support shaft 420 as a rotation axis.

When the rotary sleeve 410 rotates, the operation protrusion 411 formed on the outer circumferential surface of the rotary sleeve 410 also rotates so that the pressurized state of the operation protrusion 411 against the opening and closing cover 430 is released, so that the cover 430 is moved upwards to open the supply open area 32 of the strip cartridge 30. At the same time, the discharge plate 300 of the manipulation knob module 200 moves linearly, pushing and discharging the test strip S to the strip outlet 101. At this time, the discharge plate 300 is configured to close the supply open area 32 of the strip cartridge 30 in the process of pushing out the test strip S.

According to the structure, the opening and closing cover 430 of the cartridge opening and closing module 400 is pressed by the operation protrusion 411 of the rotary sleeve 410 so as to allow the supply open area 32 of the strip cartridge 30 to be continuously closed in a sealed manner. Only when a user manipulates the manipulation knob module 200 so as to linearly move, the opening and closing cover 430 is opened, and after the linear movement manipulation, the opening and closing cover 430 closes the supply open area 32 again in a sealed manner. Particularly, even when the manipulation knob module 200 is linearly moved in the first and second stages, the supply open area 32 is temporarily closed by the discharge plate 300, so that the sealing state for the inner space of the strip cartridge 30 can be more reliably maintained.

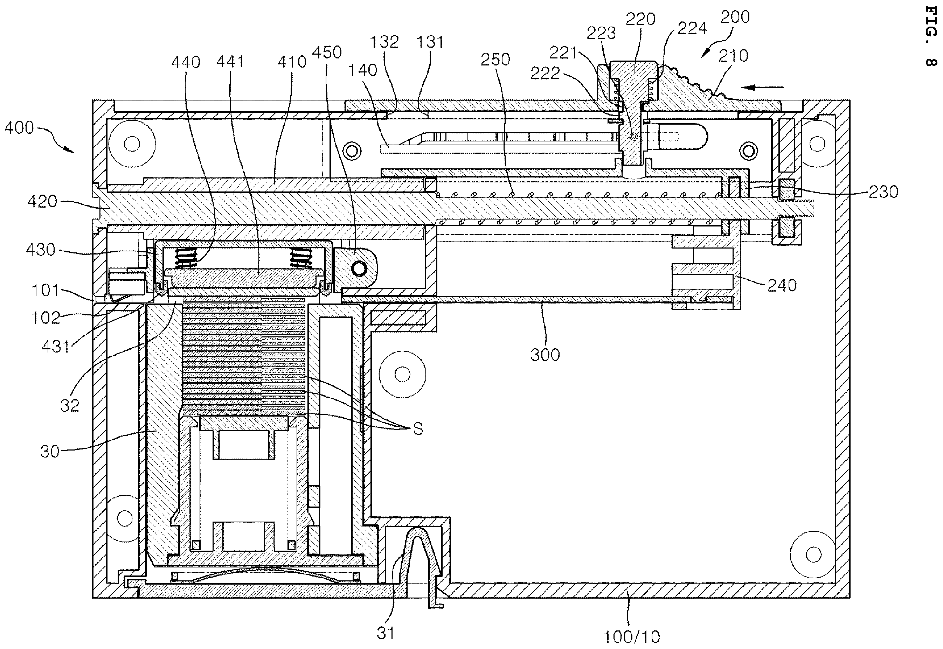

Next, the operation of the integrated blood glucose measuring device will be described with reference to FIGS. 8 to 9.

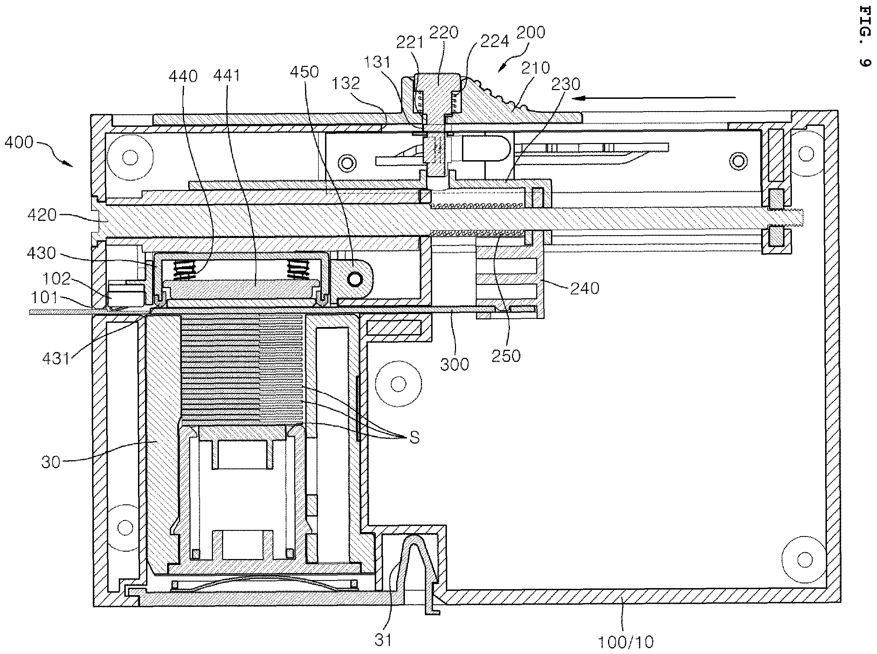

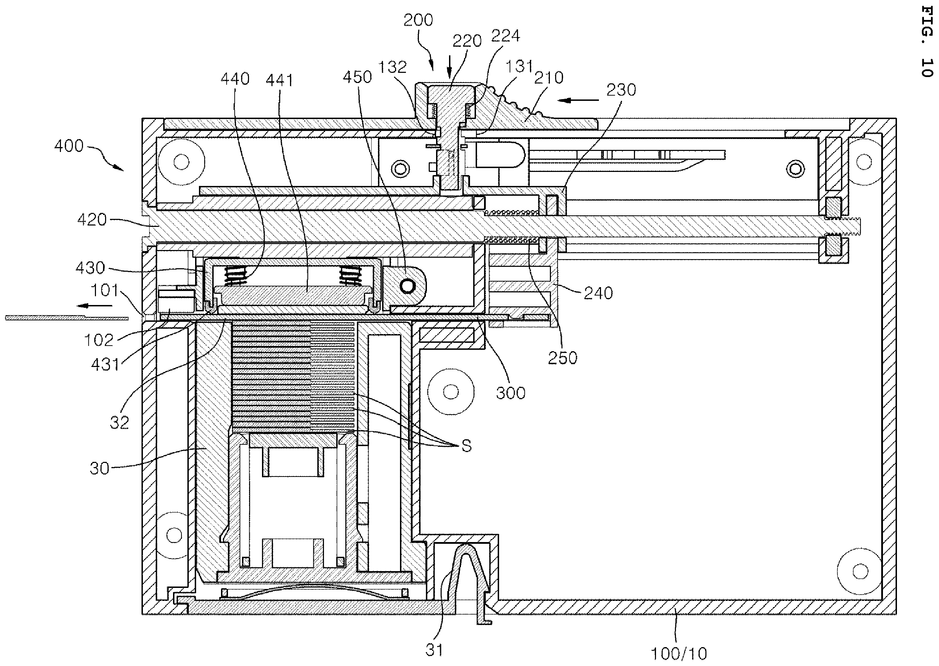

FIG. 8 is a cross-sectional view illustrating an initial movement start state of the operation knob module, FIG. 9 is a cross-sectional view illustrating a first-stage linear movement state of the operation knob module and FIG. 10 is a cross-sectional view illustrating a second-stage linear movement state of the operation knob module.

While FIG. 4 illustrates a state in which the manipulation knob module 200 is located at the reference position, FIG. 8 illustrates the initial movement start state of the manipulation knob module 200. As illustrated in FIG. 8, when the manipulation knob 210 starts to move linearly to the left with reference to the illustrated direction, the rotary sleeve 410 connected to the moving body 230 with the cam structure rotates. At the same time, the operation protrusion 411 of the rotary sleeve 410 is rotated so that the pressing state of the operating protrusion 411 against the opening and closing cover 430 is released and the opening and closing cover 430 is moved upwards by the elastic force so as to open the supply open area 32 of the strip cartridge 30. Here, the discharge plate 300 is positioned adjacent to the supply open area 32.

Thereafter, when the manipulation knob 210 is continuously moved linearly to the left, as illustrated in FIG. 9, the manipulation knob 210 is linearly moved to the distal point of the first-stage linear movement. During the first stage linear movement, the discharge plate 300 is moved to push the test strip S from the supply open area 32 of the strip cartridge 30 to the strip outlet 101. At this time, the test strip S is partially discharged to the strip outlet 101 and remains in contact with the contact pin 102 mounted around the strip outlet 101. In this state, a user can perform a blood glucose measurement operation by causing a blood sample to be adsorbed to a discharged portion of the test strip S.

Then, when the blood glucose measurement operation is completed, the second stage linear movement operation is performed by moving the manipulation knob 210 to the left while the push button 220 is maintained as being pressed down as illustrated in FIG. 10. At this stage, the discharge strip S moves and the test strip S is completely discharged and removed from the strip outlet 101.

The foregoing descriptions have been presented in order to explain certain principles of the present disclosure by way of example. A person skilled in the art to which the present invention relates could make various modifications and variations without departing from the essential features of the present invention. The foregoing embodiments disclosed herein shall be interpreted as being illustrative, while not being limitative, of the principle and scope of the present invention. It should be understood that the scope of the present invention shall be defined by the appended claims.

## Claims

1. An integrated blood glucose measuring device comprising:
a casing (10) having a structure, by which a strip cartridge (30) accommodating a plurality of test strips in an inner space thereof is interchangeably inserted into the casing, the casing (10) having a strip outlet (101) at one portion thereof, through which a test strip, among the plurality of test strips, is configured to be discharged outward; and
a strip discharging unit (20) mounted on the casing (10) to be operable by a user using a manipulation knob (210) to discharge the plurality of test strips one by one from the strip cartridge (30) through the strip outlet (101), wherein the manipulation knob (210) is configured to move linearly in first and second individual linear movements by manipulation of the user, such that during the first linear movement, the test strip is partially moved into a blood glucose measurement mode state through the strip outlet (101), and during the second linear movement, the test strip is discharged and removed from the strip outlet (101), **characterized in that:**
the strip discharging unit (20) comprises:
a manipulation knob module (200) comprising the manipulation knob (210) configured to be linearly moved by user's manipulation;
a discharge plate (300) configured to be linearly moved and integrated with the manipulation knob module (200) to push and discharge the test strip, which is supplied through a supply open area (32) of the strip cartridge (30), to the strip outlet (101); and
a cartridge opening/closing module (400) configured to open and close the supply open area (32) of the strip cartridge (30) in association with the linear movement of the manipulation knob module (200),
wherein the cartridge opening/closing module (400) comprises:
a rotary sleeve (410) configured to rotate about a longitudinal axis integrally with the linear movement of the manipulation knob module (200), with an operation protrusion (411) integrally provided on one portion of an outer circumference thereof; and
an opening/closing cover (430) arranged to be movable up and down linearly to open/close the supply open area (32) of the strip cartridge and elastically supported by an elastic spring (440) in a direction to contact the rotary sleeve (410),
wherein, as the rotary sleeve (410) rotates, the opening/closing cover (430) is pressed by or released from the operation protrusion (411) to move up and down by an elastic force.

2. The integrated blood glucose measuring device according to claim 1, wherein the strip discharging unit (20) is configured such that the manipulation knob (210) can be returned to an original position thereof after both the first and second linear movements are performed.

3. The integrated blood glucose measuring device according to claim 1, wherein the manipulation knob module (200) is exposed to one surface of the casing (10) to be coupled in a linearly movable manner,
wherein the manipulation knob module (200) comprises:
a push button (220) coupled to the manipulation knob (210) to be elastically supported upward by the manipulation knob in a direction perpendicular to the linear movement direction of the manipulation knob, so as to linearly move integrally with the manipulation knob; and
a moving body (230) coupled to the manipulation knob (210) or the push button (220) to linearly move integrally with the manipulation knob or the push button,
wherein the casing (10) is provided with a separation movement guide (130) along which the push button (220) is guided by the manipulation know module (200) to linearly move in the first and second linear movements.

4. The integrated blood glucose measuring device according to claim 3, wherein the separation movement guide (130) is configured to guide the manipulation knob module (200) to move linearly in the first and second linear movement such that the second linear movement is performed while the push button (220) is pressed downward.

5. The integrated blood glucose measuring device according to claim 4, wherein the casing (10) is provided with a movement restricting guide (140) configured to guide the manipulation knob module (200) to prevent the manipulation knob module from returning to a reference position before the first and second linear movements are completed.

6. The integrated blood glucose measuring device according to claim 5, wherein the movement restricting guide (140) is provided to restrict the manipulation knob module (200) from returning to the original position in a state in which the first linear movement of the manipulation knob module is completed.

7. The integrated blood glucose measuring device according to claim 5, wherein the movement restricting guide (140) comprises:
a movement path guide rail (141) configured to guide the first and second linear movement paths of the manipulation knob module (200); and
a return path guide rail (142) configured to guide an original return path of the manipulation knob module (200),
wherein the movement path guide rail is provided with a ratchet protrusion (141-1) to prevent the manipulation knob module (200) from returning to the original position.

8. The integrated blood glucose measuring device according to claim 7, wherein the manipulation knob module (200) comprises:
a push button (220) coupled to the manipulation knob (210) to be elastically supported upward by the manipulation knob in a direction perpendicular to the linear movement direction of the manipulation knob, so as to linearly move integrally with the manipulation knob; and
a moving body (230) coupled to the manipulation knob (210) or the push button (220) to be linearly moved at the same time,
wherein the rotary sleeve (410) of the cartridge opening/closing module (400) and the moving body (230) of the manipulation knob module (200) are provided with a cam protrusion (231) and a cam groove (412) mutually engaged with a cam structure, wherein the rotary sleeve (410) is configured to rotate through the cam structure as the moving body (230) moves linearly.

9. The integrated blood glucose measuring device according to claim 1, wherein, in a state in which the cartridge opening/closing module (400) opens the supply open area (32) of the strip cartridge (30), the discharge plate (300) moves linearly to push out the test strip so as to close the supply open area of the strip cartridge.

## Patentansprüche

1. Integrierte Blutzuckermessvorrichtung, umfassend:
ein Gehäuse (10), das eine Struktur aufweist, anhand derer eine Streifenkartusche (30), die eine Vielzahl von Teststreifen in einem Innenraum davon beherbergt, austauschbar in das Gehäuse eingesetzt wird, wobei das Gehäuse (10) an einem Abschnitt davon einen Streifenauslass (101) aufweist, durch den ein Teststreifen von der Vielzahl von Teststreifen konfiguriert ist, nach außen abgegeben zu werden; und
eine Streifenabgabeeinheit (20), die an dem Gehäuse (10) angebracht ist, um von einem Benutzer unter Verwendung eines Bedienknopfes (210) betätigbar zu sein, um die Vielzahl von Teststreifen nacheinander durch den Streifenauslass (101) aus der Streifenkartusche (30) abzugeben, wobei der Bedienknopf (210) konfiguriert ist, um sich durch Bedienung des Benutzers linear in ersten und zweiten individuellen linearen Bewegungen zu bewegen, sodass der Teststreifen während der ersten linearen Bewegung teilweise durch den Streifenauslass (101) in einen Blutzuckermessmodus-Zustand bewegt wird und der Teststreifen während der linearen Bewegung aus dem Streifenauslass (101) abgegeben und entfernt wird, **dadurch gekennzeichnet, dass:**
die Streifenabgabeeinheit (20) Folgendes umfasst:
ein Bedienknopfmodul (200), das den Bedienknopf (210) umfasst, der konfiguriert ist, um durch Bedienung des Benutzers linear bewegt zu werden;
eine Abgabeplatte (300), die konfiguriert ist, um linear bewegt und in das Bedienknopfmodul (200) integriert zu werden, um den Teststreifen, der durch eine offene Zuführfläche (32) der Streifenkartusche (30) zugeführt wird, zu dem Streifenauslass (101) zu drücken und abzugeben; und
ein Kartuschenöffnungs-/Kartuschenschließmodul (400), das konfiguriert ist, um die offene Zuführfläche (32) der Streifenkartusche (30) in Verbindung mit der linearen Bewegung des Bedienknopfmoduls (200) zu öffnen und zu schließen,
wobei das Kartuschenöffnungs-/Kartuschenschließmodul (400) Folgendes umfasst:
eine Drehhülse (410), die konfiguriert ist, um sich einstückig mit der linearen Bewegung des Bedienknopfmoduls (200) um eine Längsachse zu drehen, wobei auf einem Abschnitt eines Außenumfangs davon einstückig ein Betriebsvorsprung (411) bereitgestellt ist; und
eine Öffnungs-/Schließabdeckung (430), die angeordnet ist, um linear zu dem Öffnen/Schließen der offenen Zuführfläche (32) der Streifenkartusche nach oben und unten bewegbar zu sein, und von einer elastischen Feder (440) in einer Richtung elastisch gestützt wird, um mit der Drehhülse (410) in Kontakt zu treten,
wobei, wenn sich die Drehhülse (410) dreht, die Öffnungs-/Schließabdeckung (430) durch den Betriebsvorsprung (411) zusammengedrückt oder von ihm gelöst wird, um sich durch eine elastische Kraft nach oben und unten zu bewegen.

2. Integrierte Blutzuckermessvorrichtung nach Anspruch 1, wobei die Streifenabgabeeinheit (20) so konfiguriert ist, dass der Bedienknopf (210) in seine Originalposition zurückversetzt werden kann, nachdem sowohl die erste als auch die zweite lineare Bewegung erfolgt sind.

3. Integrierte Blutzuckermessvorrichtung nach Anspruch 1, wobei das Bedienknopfmodul (200) einer Oberfläche des Gehäuses (10) ausgesetzt ist, um in einer linear bewegbaren Art gekoppelt zu werden,
wobei das Bedienknopfmodul (200) Folgendes umfasst:
eine Drucktaste (220), die mit dem Bedienknopf (210) gekoppelt ist, um von dem Bedienknopf aufwärts in einer Richtung, die zu der linearen Bewegungsrichtung des Bedienknopfes senkrecht ist, elastisch gestützt zu werden, um sich einstückig mit dem Bedienknopf linear zu bewegen; und
einen sich bewegenden Körper (230), der mit dem Bedienknopf (210) oder der Drucktaste (220) gekoppelt ist, um sich einstückig mit dem Bedienknopf oder der Drucktaste linear zu bewegen,
wobei das Gehäuse (10) mit einer Trennbewegungsführung (130) bereitgestellt ist, entlang derer die Drucktaste (220) von dem Bedienknopfmodul (200) geführt wird, um sich in der ersten und zweiten linearen Bewegung linear zu bewegen.

4. Integrierte Blutzuckermessvorrichtung nach Anspruch 3, wobei die Trennbewegungsführung (130) konfiguriert ist, um das Bedienknopfmodul (200) so zu führen, dass es sich in der ersten und zweiten linearen Bewegung linear bewegt, sodass die zweite lineare Bewegung durchgeführt wird, während die Drucktaste (220) heruntergedrückt wird.

5. Integrierte Blutzuckermessvorrichtung nach Anspruch 4, wobei das Gehäuse (10) mit einer bewegungseinschränkenden Führung (140) bereitgestellt ist, die konfiguriert ist, um das Bedienknopfmodul (200) zu führen, um zu verhindern, dass das Bedienknopfmodul in eine Referenzposition zurückkehrt, bevor die erste und die zweite lineare Bewegung abgeschlossen sind.

6. Integrierte Blutzuckermessvorrichtung nach Anspruch 5, wobei die bewegungseinschränkende Führung (140) bereitgestellt ist, um das Bedienknopfmodul (200) darin einzuschränken, in einem Zustand in die Originalposition zurückzukehren, in dem die erste lineare Bewegung des Bedienknopfmoduls abgeschlossen ist.

7. Integrierte Blutzuckermessvorrichtung nach Anspruch 5, wobei die bewegungseinschränkende Führung (140) Folgendes umfasst:
eine Bewegungsweg-Führungsschiene (141), die konfiguriert ist, um den ersten und zweiten linearen Bewegungsweg des Bedienknopfmoduls (200) zu führen; und
eine Rückweg-Führungsschiene (142), die konfiguriert ist, um einen Originalrückweg des Bedienknopfmoduls (200) zu führen,
wobei die Bewegungsweg-Führungsschiene mit einem Ratschenvorsprung (141-1) bereitgestellt ist, um zu verhindern, dass das Bedienknopfmodul (200) in die Originalposition zurückkehrt.

8. Integrierte Blutzuckermessvorrichtung nach Anspruch 7, wobei das Bedienknopfmodul (200) Folgendes umfasst:
eine Drucktaste (220), die mit dem Bedienknopf (210) gekoppelt ist, um von dem Bedienknopf aufwärts in einer Richtung, die zu der linearen Bewegungsrichtung des Bedienknopfes senkrecht ist, elastisch gestützt zu werden, um sich einstückig mit dem Bedienknopf linear zu bewegen; und
einen sich bewegenden Körper (230), der mit dem Bedienknopf (210) oder der Drucktaste (220) gekoppelt ist, um sich gleichzeitig linear zu bewegen,
wobei die Drehhülse (410) des Kartuschenöffnungs-/Kartuschenschließmoduls (400) und der sich bewegende Körper (230) des Bedienknopfmoduls (200) mit einem Nockenvorsprung (231) und einer Kurvennut (412) bereitgestellt sind, die wechselseitig in eine Nockenstruktur eingerastet sind, wobei die Drehhülse (410) konfiguriert ist, um sich durch die Nockenstruktur zu drehen, wenn der sich bewegende Körper (230) sich linear bewegt.

9. Integrierte Blutzuckermessvorrichtung nach Anspruch 1, wobei sich die Abgabeplatte (300) in einem Zustand, in dem das Kartuschenöffnungs-/Kartuschenschließmodul (400) die offene Zuführfläche (32) der Streifenkartusche (30) öffnet, linear bewegt, um den Teststreifen herauszudrücken, um die offene Zuführfläche der Streifenkartusche zu schließen.

## Revendications

1. Dispositif intégré de mesure de glycémie comprenant :
un boîtier (10) présentant une structure, par laquelle une cartouche de bandes (30) logeant une pluralité de bandes-test dans un espace intérieur de celle-ci est insérée de manière interchangeable dans le boîtier, le boîtier (10) présentant une sortie de bande (101) au niveau d'une partie de celui-ci, par laquelle une bande-test, parmi la pluralité de bandes-test, est configurée pour être sortie vers l'extérieur ; et
une unité de sortie de bande (20) montée sur le boîtier (10) pour pouvoir être actionnée par un utilisateur à l'aide d'un bouton de manipulation (210) pour faire sortir la pluralité de bandes-test une par une de la cartouche de bandes (30) par la sortie de bande (101), dans lequel le bouton de manipulation (210) est configuré pour se déplacer linéairement selon des premier et second déplacements linéaires individuels par manipulation de l'utilisateur, de telle sorte que, durant le premier déplacement linéaire, la bande-test est partiellement déplacée dans un état de mode de mesure de glycémie par la sortie de bande (101), et durant le second déplacement linéaire, la bande-test est sortie et retirée de la sortie de bande (101), **caractérisé en ce que** :
l'unité de sortie de bande (20) comprend :
un module de bouton de manipulation (200) comprenant le bouton de manipulation (210) configuré pour être déplacé linéairement par une manipulation de l'utilisateur ;
une plaque de sortie (300) configurée pour être déplacée linéairement et intégrée au module de bouton de manipulation (200) pour pousser et faire sortir la bande-test, qui est apportée par le biais d'une zone ouverte d'alimentation (32) de la cartouche de bandes (30), vers la sortie de bande (101) ; et
un module d'ouverture/fermeture de cartouche (400) configuré pour ouvrir et fermer la zone ouverte d'alimentation (32) de la cartouche de bandes (30) en association avec le déplacement linéaire du module de bouton de manipulation (200),
dans lequel le module d'ouverture/fermeture de cartouche (400) comprend :
un manchon rotatif (410) configuré pour tourner autour d'un axe longitudinal d'un seul tenant avec le déplacement linéaire du module de bouton de manipulation (200), avec une saillie d'actionnement (411) disposée d'un seul tenant sur une partie d'une circonférence extérieure de celui-ci ; et
un cache d'ouverture/fermeture (430) agencé pour pouvoir monter et descendre linéairement pour ouvrir/fermer la zone ouverte d'alimentation (32) de la cartouche de bandes et supporté élastiquement par un ressort élastique (440) dans une direction pour entrer en contact avec le manchon rotatif (410),
dans lequel, quand le manchon rotatif (410) tourne, le cache d'ouverture/fermeture (430) est pressé par la saillie d'actionnement (411) ou libéré de cette dernière pour monter et descendre par une force élastique.

2. Dispositif intégré de mesure de glycémie selon la revendication 1, dans lequel l'unité de sortie de bande (20) est configurée de telle sorte que le bouton de manipulation (210) peut être ramené dans sa position d'origine après que les premier et second déplacements linéaires ont tous les deux été effectués.

3. Dispositif intégré de mesure de glycémie selon la revendication 1, dans lequel le module de bouton de manipulation (200) est exposé sur une surface du boîtier (10) pour être accouplé d'une manière linéairement mobile,
dans lequel le module de bouton de manipulation (200) comprend :
un bouton-poussoir (220) accouplé au bouton de manipulation (210) pour être élastiquement supporté vers le haut par le bouton de manipulation dans une direction perpendiculaire à la direction de déplacement linéaire du bouton de manipulation, de manière à se déplacer linéairement d'un seul tenant avec le bouton de manipulation ; et
un corps mobile (230) accouplé au bouton de manipulation (210) ou au bouton-poussoir (220) pour se déplacer linéairement d'un seul tenant avec le bouton de manipulation ou le bouton-poussoir,
dans lequel le boîtier (10) est pourvu d'un guide de déplacement de séparation (130) le long duquel le bouton-poussoir (220) est guidé par le module de bouton de manipulation (200) pour se déplacer linéairement dans les premier et second déplacements linéaires.

4. Dispositif intégré de mesure de glycémie selon la revendication 3, dans lequel le guide de déplacement de séparation (130) est configuré pour guider le module de bouton de manipulation (200) pour se déplacer linéairement dans les premier second déplacements linéaires de telle sorte que le second déplacement linéaire est effectué alors que le bouton-poussoir (220) est pressé vers le bas.

5. Dispositif intégré de mesure de glycémie selon la revendication 4, dans lequel le boîtier (10) est pourvu d'un guide de limitation de déplacement (140) configuré pour guider le module de bouton de manipulation (200) pour empêcher le module de bouton de manipulation de revenir à une position de référence avant que les premier et second déplacements linéaires ne soient terminés.

6. Dispositif intégré de mesure de glycémie selon la revendication 5, dans lequel le guide de limitation de déplacement (140) est prévu pour empêcher le module de bouton de manipulation (200) de revenir à la position d'origine dans un état dans lequel le premier déplacement linéaire du module de bouton de manipulation est terminé.

7. Dispositif intégré de mesure de glycémie selon la revendication 5, dans lequel le guide de limitation de déplacement (140) comprend :
un rail (141) de guide de limitation de déplacement configuré pour guider les premier et second chemins de déplacement linéaire du module de bouton de manipulation (200) ; et
un rail (142) de guide de chemin de retour configuré pour guider un chemin de retour à l'origine du module de bouton de manipulation (200),
dans lequel le rail de guide de chemin de déplacement est pourvu d'une saillie à cliquet (141-1) pour empêcher le module de bouton de manipulation (200) de revenir à la position d'origine.

8. Dispositif intégré de mesure de glycémie selon la revendication 7, dans lequel le module de bouton de manipulation (200) comprend :
un bouton-poussoir (220) accouplé au bouton de manipulation (210) pour être supporté de manière élastique vers le haut par le bouton de manipulation dans une direction perpendiculaire à la direction de déplacement linéaire du bouton de manipulation, de manière à se déplacer linéairement d'un seul tenant avec le bouton de manipulation ; et
un corps mobile (230) accouplé au bouton de manipulation (210) ou au bouton-poussoir (220) pour être déplacé linéairement en même temps,
dans lequel le manchon rotatif (410) du module d'ouverture/fermeture de cartouche (400) et le corps mobile (230) du module de bouton de manipulation (200) sont pourvus d'une saillie de came (231) et d'une rainure de came (412) mutuellement en prise avec une structure de came, dans lequel le manchon rotatif (410) est configuré pour tourner à travers la structure de came quand le corps mobile (230) se déplace linéairement.

9. Dispositif intégré de mesure de glycémie selon la revendication 1, dans lequel, dans un état dans lequel le module d'ouverture/fermeture de cartouche (400) ouvre la zone ouverte d'alimentation (32) de la cartouche de bandes (30), la plaque de sortie (300) se déplace linéairement pour pousser dehors la bande-test de manière à fermer la zone ouverte d'alimentation de la cartouche de bandes.
